# EUROPÄISCHE PATENTSCHRIFT

(11) **EP 1 681 061 B1**
(45) Veröffentlichungstag und Bekanntmachung des Hinweises auf die Patenterteilung: **20.03.2013**
(21) Anmeldenummer: 06000495.9
(22) Anmeldetag: 11.01.2006
(51) Int. Cl.: A61K 36/537, A61K 36/79, A61K 33/10, A61P 43/00

(54) **Zusammensetzung zur Behandlung der Apnoe, des Schnarchens und von Schlafstörungen**
Composition for treating apnea, snoring and sleep disorders
Composition pour le traitement de l'apnée, du ronflement et des troubles du sommeil

(30) Priorität: 11.01.2005 DE 102005001252
(43) Veröffentlichungstag der Anmeldung: 19.07.2006
(73) Patentinhaber: Jobes Vital GmbH, 50735 Köln (DE)
(72) Erfinder: Schembra, Bettina, 78052 Villingen-Schwenningen (DE); Schembra, Jörg, 78052 Villingen-Schwenningen (DE)
(74) Vertreter: Isarpatent

(56) Entgegenhaltungen:
- WO-A-00/57726
- GB-A- 898 659
- GB-A- 1 021 924
- DATABASE WPI Week 200403 Derwent Publications Ltd., London, GB; AN 2004-023820 XP002381580 "Sedative Jejube Kernel Capsule" & CN 1 435 206 A (XIANLING PHARMACEUTICAL CO., LTD., GUIZHOU) 13. August 2003 (2003-08-13)
- WING Y K: "Herbal treatment of insomnia" HONG KONG MEDICAL JOURNAL 2001 HONG KONG, Bd. 7, Nr. 4, 2001, Seiten 392-402, XP002381244 ISSN: 1024-2708
- SUN X ET AL: "Magnesium as NMDA receptor blocker in the traditional Chinese medicine Danshen" PHYTOMEDICINE, GUSTAV FISCHER VERLAG, STUTTGART, DE, Bd. 12, Nr. 3, 22. März 2005 (2005-03-22), Seiten 173-177, XP004957232 ISSN: 0944-7113

## Beschreibung

Die vorliegende Erfindung betrifft eine Zusammensetzung, die Salvia Miltiorrhizae oder Pflanzenteile hiervon, Fructus Schizandrae oder Pflanzenteile hiervon und ein oder mehrere Calcium- und/oder Magnesiumsalze umfasst und zur Behandlung der Schlafapnoe, krankhaften Formen des Schnarchens und von durch Schnarchen bedingten Schlafstörungen eingesetzt werden kann. Die vorliegende Erfindung umfasst weiterhin ein Verfahren zur Herstellung dieser Zusammensetzung.

Beschwerden im Zusammenhang mit nicht erholsamem Schlaf oder nicht ausreichendem Schlaf können unter anderem durch Apnoe und Schnarchen hervorgerufen werden. Nicht erholsamer Schlaf kann zu Tagesmüdigkeit und damit verbunden zu verminderter Leistungsfähigkeit und schließlich zu einem Verlust an Lebensqualität führen. Insbesondere das Schnarchen stellt nicht nur für den Betroffenen selbst, sondern auch für andere Personen im privaten Umfeld eine ernstzunehmende Einschränkung der Lebensqualität dar.

In früheren Klassifikationsschemata organischer oder psychiatrischer Erkrankungen, wie z.B. dem ICD-9, sucht man vergeblich den Terminus nicht erholsamer Schlaf. Erst modernere diagnostische Schemata, wie etwa DSM-III-R bzw. DSM-IV in der Psychiatrie oder die ICSD-R für den Bereich der Schlafstörungen, verwenden den Begriff ,,nicht erholsamer Schlaf". Im DSM-IV werden bei den Kriterien der primären Insomnie die Beschwerden Ein- oder Durchschlafstörungen und nicht erholsamer Schlaf aufgezählt (engl.: *"non restorative sleep*"). Auch die ICSD-R definiert Insomnie als "*Beschwerde ungenügenden Schlafes oder sich nicht erholt zu fühlen nach der üblichen Schlafzeit*".

Die Frage der Krankheitswertigkeit des nicht erholsamen Schlafs ergibt sich aus seinen Konsequenzen: die ICSD teilt die Insomnie bzw. die Schläfrigkeit nach dem Schweregrad in leicht, mittelschwer und schwer ein. Der nicht erholsame Schlaf bei leichter, mittelschwerer und schwerer Insomnie führt zu graduell unterschiedlichen Beeinträchtigungen der sozialen und beruflichen Leistungsfähigkeit und ist mit Unruhegefühlen, Reizbarkeit, Angst, Depressivität, Erschöpfung und Müdigkeit verbunden. Der nicht erholsame Schlaf bei leichter, mittelschwerer und schwerer Schläfrigkeit tagsüber (Hypersomnie) führt zu graduell unterschiedlicher Vigilanzbeeinträchtigung bzw. Schlafepisoden tagsüber, die in starkem Maß mit der sozialen oder beruflichen Leistungsfähigkeit der Betroffenen interferieren und zudem bei bestimmten Krankheitsbildern die körperliche Gesundheit nachhaltig beeinträchtigen. Der Terminus nicht erholsamer Schlaf eignet sich somit gut als grundlegende Beschwerde, die allen Dyssomnien nach Beschreibung durch die ICSD gemein ist (siehe Leitlinien der Deutschen Gesellschaft für Schlafforschung und Schlafmedizin (DGSM), August 2001).

Eine Ursache für nicht erholsamen Schlaf oder allgemein für eine Schlafstörung sind schlafbezogene Atmungsstörungen, insbesondere Schnarchen und Apnoe.

In epidemiologischer Hinsicht werden in den USA gegenwärtig mehrere groß angelegte Studien durchgeführt, die sich intensiv mit der Häufigkeit schlafbezogener Atemstörungen in der Allgemeinbevölkerung und deren Konsequenzen befassen. Es handelt sich hierbei um die Cardiovascular Health Study, die Wisconsin Sleep Cohort Study sowie die Sleep Heart Health Study. Bisher sprechen die Ergebnisse dieser Studien dafür, dass Schnarchen und vom Bettpartner beobachtete Atempausen in der Allgemeinbevölkerung häufig auftreten. Die berichtete Tagesschläfrigkeit betraf 10 - 20% der Allgemeinbevölkerung.

In der Wisconsin Sleep Cohort Study konnte eine Prävalenz des obstruktiven Schlaf-Apnoe-Syndroms, definiert durch einen Apnoe-Hypopnoe-Index 15/Std., bei ca. 10% der repräsentativen Stichprobe festgestellt werden.

Eine neue Studie aus der Arbeitsgruppe von Ohayon, die mit dem SLEEP-EVAL (standardisiertes Interview) durchgeführt wurde, fand in Deutschland eine Prävalenz der obstruktiven Schlaf-Apnoe von 1,8%.

Der Begriff Apnoe bedeutet medizinisch Aussetzer der Atmung während des Schlafes. Diese Atemaussetzer können zu Tagesschläfrigkeit führen und der Auslöser für Bluthochdruck, Herzversagen sowie Herz- und Schlaganfälle sein. Die Schlafapnoe wird vielfach durch lautes Schnarchen angedeutet. In schweren Fällen kann die Atmung bis zu 75% der gesamten Schlafdauer aussetzen. Der gestörte Nachtschlaf kann extreme Tagesschläfrigkeit verursachen und zu gravierenden Belastungen führen. Schlafapnoe kann zu Konzentrationsstörungen, Vergesslichkeit, Zerstreutheit, Angstzuständen und Depressionen führen. Auch die Umgebung kann durch die lauten Schnarchgeräusche, die eine Lautstärke erreichen können, die an die Geräuschentwicklung von Pressluftbohrern heranreicht, gestört werden.

Es wird zwischen zwei verschiedenen Formen der Schlafapnoe unterschieden. Bei der obstruktiven Schlafapnoe liegt eine Obstruktion der oberen Atemwege vor. Bei der zentralen Schlafapnoe bleiben die Atemwege zwar geöffnet, aber die Muskeln in Brust und Zwerchfell sind nicht aktiv.

Das obstruktive Schlafapnoesyndrom (OSAS) ist gekennzeichnet durch periodisch wiederkehrende Obstruktionen der oberen Atemwege, die während des Schlafes auftreten und gewöhnlich eine alveoläre Minderbelüftung und konsekutiv ein Absinken des Sauerstoffgehaltes und ein Ansteigen der CO₂-Konzentration im Blut zur Folge haben. Vom obstruktiven Schlafapnoesyndrom ist mindestens 1% der Gesamtbevölkerung betroffen, überwiegend die Altersgruppe der 40- bis 65jährigen Männer.

Das zentrale Schlafapnoesyndrom ist gekennzeichnet durch repetitiven Stillstand der Atmung oder Unterbleiben der ventilatorischen Anstrengungen im Schlaf, gewöhnlich verbunden mit Sauerstoffentsättigung.

Unter den Parasomnien eingeordnet findet sich das primäre Schnarchen (ICSD-Code 780.53-1). Es ist definiert als einfaches Schnarchen ohne Schlafapnoe und ohne die Symptome und Folgeerscheinungen von obstruktivem Schnarchen und obstruktiven Apnoen. Mithin handelt es sich hier um ein Schnarchen ohne signifikante Schädigungen von Atmung und Herz-Kreislaufsystem und ohne Beeinträchtigung der Schlafqualität. Symptomatisch hingegen ist morgendliche Mundtrockenheit. Der weit überwiegende Teil der schnarchenden Menschen weist das sog. primäre Schnarchen auf. Es wird z.B. für 50jährige Männer in ca. 50% der Fälle berichtet.

Zur Behandlung von atmungsbedingten Schlafstörungen kommen bisher unterschiedliche Verfahren zum Einsatz. Bei der kontinuierlichen positiven Überdruckbeatmung, der CPAP-Therapie (Continuous positive airway pressure), werden die Patienten über individuell angepasste Nasenmasken mit Raumluft beatmet. Dabei muss stets die gesamte Nacht mit der Nasenmaske verbracht werden. Der Gebrauch des Gerätes wird jedoch von vielen Patienten als zu lästig empfunden. Bei manchen Patienten kommen individuell angepasste Aufbissschienen für Ober- und Unterkiefer zum Einsatz. Mit diesem technischen Hilfsmittel sollen die Atemwege offen gehalten werden, indem Kiefer, Zunge und weicher Gaumen leicht nach vorne verlagert werden. Die Schienen sind jedoch oft recht teuer und wirken nur teilweise. Auch lässt sich nicht mit Sicherheit vorhersagen, ob diese Schienen zum Erfolg verhelfen.

Bei physischen Anomalien, die die Atmung im Schlaf ungünstig beeinflussen, können in manchen Fällen chirurgische Korrekturen vorgenommen werden. Zu diesen Anomalien zählen beispielsweise vergrößerte Mandeln und Polypen, sowie Missbildungen des Kiefers und eine für die Atmung ungünstig verlaufende Nasenscheidewand. Bei schweren lebensbedrohlichen Apnoesyndromen wird gelegentlich auch die Dracheotomie (Luftröhrenschnitt) eingesetzt. Diese Therapieverfahren stellen jedoch nur die allerletzte Notfallmaßnahme dar.

Der Einsatz von Medikamenten sollte stets mit äußerster Vorsicht vorgenommen werden. So können sich zum Beispiel zentralwirksame Medikamente gegen Kopfschmerzen und Angstzustände auf Schlaf und Atmung negativ auswirken. Schlafmittel sollten ebenfalls gemieden werden, da auch sie die Atmungsaktivität herabsetzen und Schlafapnoe begünstigen können.

### <Seite 4a>

Bisher profitieren Schlafapnoeiker nur in geringem Maße von einer medikamentösen Behandlung. Ein wirklich gegen schlafbezogene Atmungsstörungen wie Schnarchen oder Schlafapnoe wirkendes Medikament wurde bisher noch nicht gefunden.

Es ist daher eine Aufgabe der vorliegenden Erfindung, eine Zusammensetzung bereitzustellen, die zur weitestgehend risikolosen Behandlung der Schlafapnoe, krankhaften Formen des Schnarchens und von durch Schnarchen bedingten Schlafstörungen eingesetzt werden kann.

WO00/57727 offenbart Nutraceuticals, die unter anderem Salvia Miltiorrhizae, Fructus Schizandrae und Mineralien (Erdalkalimetallsalze) enthalten können.

Aus CN-A-1 435 206 ist der Einsatz von Kapseln, die unter anderem Salvia Miltiorrhizae und Fructus Schizandrae enthalten, für medizinische Zwecke bekannt. Hier wird jedoch nicht beschrieben, dass die Zusammensetzung Erdalkalimetalle als wirksame Bestandteile enthält. Diese Aufgabe wird vom Gegenstand der unabhängigen Ansprüche gelöst. Bevorzugte Ausführungsformen sind in den abhängigen Ansprüchen angegeben.

Erfindungsgemäß stellt die Zusammensetzung nach Anspruch 1 eine solche Zusammensetzung zur Behandlung der Schlafapnoe, krankhafter Formen des Schnarchens und von durch Schnarchen bedingter Schlafstörungen bereit.

Gemäß eines ersten Aspektes betrifft die Erfindung somit eine Zusammensetzung, die Salvia Miltiorrhizae oder Pflanzenteile hiervon, Fructus Schizandrae oder Pflanzenteile hiervon und ein oder mehrere Erdalkalimetallsalze als wirksame Bestandteile umfasst, wobei als Erdalkalimetallsalze Calcium- und/oder Magnesiumsalze, vorzugsweise Calcium- und/oder Magnesiumcarbonat, eingesetzt werden.

Der Ausdruck "wirksame Bestandteile" bedeutet hier, dass die einzelnen Komponenten in solchen Mengen enthalten sind, die zur Erzielung der therapeutischen Wirkung geeignet sind. Dies betrifft insbesondere auch die in der Zusammensetzung enthaltenen Erdalkalimetallsalze, die ebenfalls einen wirksamen Bestandteil darstellen.

Erdalkalimetallsalze, z.B. Calcium- oder Magnesiumsalze, werden in der pharmazeutischen Technologie zwar auch häufig als Hilfsmittel verwendet, beispielsweise als Füllstoffe etc. Hierbei kommen sie aber beispielsweise in Mengen von nur 0,5% (G/G) zum Einsatz, was für die Erzielung einer therapeutischen Wirkung bei weitem zu niedrig ist.

Es hat sich überraschenderweise herausgestellt, dass eine Kombination der drei Bestandteile eine hervorragende Wirkung zur Behandlung der Schlafapnoe, krankhaften Formen des Schnarchens und von durch Schnarchen bedingten Schlafstörungen aufweist.

Diese Erkenntnis war unerwartet, da die (hinlänglich bekannten und auch medizinisch eingesetzten) Einzelbestandteile der Zusammensetzung eine solche Wirkung nicht zeigen.

Vom ersten Bestandteil, Salvia Miltiorrhizae, kommt bevorzugt die Wurzel (Radix) zum Einsatz. Der chinesische Name der Radix Salvia Miltiorrhizae ist DAN SHEN. In der chinesischen Medizin gehört DAN SHEN zu den blutbelebenden Drogen. DAN SHEN wirkt thrombolytisch, antihypertensiv, sedativ, antimikrobiell, antipyretisch/antiinflammatorisch und hepatoprotektiv, verbessert die periphere Durchblutung und senkt den Gefäßwiderstand.

Der entsprechende englische Name ist "Salvia Root" (Salbeiwurzel). Die Pflanze gehört zur Familie der Labiatae (Lippenblütler) (siehe auch "Chinese Medical Herbology and Pharmacology", John K. Chen, Tina T. Chen, Art of Medicine Press, Inc., CA, USA, 2004; "Grosses Handbuch der chinesischen Phytotherapie, Akupunktur und Diätetik", S. Englert, Verlag für ganzheitliche Medizin, Kötzting).

Fructus Schizandrae, auch Spaltkölbchen genannt, ist nach der chinesischen Medizin eine stabilisierende und haltende Droge. Sie enthält unter anderem Lignane, Vitamin C und E. Die isolierten Lignane (Schizandrin, Schizandrol) wirken hepatoprotektiv, als Radikalfänger, Leberregenerations-fördernd, aber auch antiinflammatorische, tumorhemmende, neuroleptische und antikonvulsive Eigenschaften wurden nachgewiesen. Schizandrae Fructus, dessen chinesischer Name WU WEI ZI ist, kann zur Verbesserung der Leberfunktion, bei allergischen Hauterscheinungen sowie bei Hypertonie und Neurasthenie eingesetzt werden. In Tierexperimenten wurde bei Überdosierung Ruhelosigkeit, Insomnie und Dyspnoe beobachtet.

Als Erdalkalimetallsalze werden Calcium- und/oder Magnesiumsalze eingesetzt, hier insbesondere deren Carbonate. Als besonders vorteilhaft hat sich die Zusammensetzung der Magnesium- und Calciumsalze im Dolomit erwiesen. Das gemahlene Dolomitpulver kann z. B. lebensmittelrein in den Berchtesgadener Alpen gewonnen werden. Es ist bekannt, dass Dolomitkalk die Muskulatur entspannt.

Reiner Dolomit enthält etwa 45,7% Magnesiumcarbonat und 54,3% Calciumcarbonat. Dieser kann z. B. von der Firma Schöndorfer Dolomitwerk, Oberjettenberg, bezogen werden.

Die Kombination der Wirkstoffe von Salvia Miltiorrihizae und Fructus Schizandrae mit Magnesium- und/oder Calciumsalzen zeigt eine unerwartete Wirkung bei Schlafapnoe, krankhafter Form des Schnarchen und von durch Schnarchen bedingter Schlafstörungen. Dies mag umso mehr deswegen erstaunen, da - wie oben angesprochen - Fructus Schizandrae bei Überdosierung Ruhelosigkeit, Insomnie und Dyspnoe verursacht.

In einer bevorzugten Ausführungsform wird ein Anteil an Salvia Miltiorrhizae oder deren Pflanzenteilen im Bereich von 40-80 Gew.-%, bevorzugt von 50-70 Gew.-%, und besonders bevorzugt von 60 Gew.-% eingesetzt.

In einer weiteren bevorzugten Ausführungsform beträgt der Anteil an Fructus Schizandrae oder deren Pflanzenteilen von 10-50 Gew.-%, bevorzugt von 20-40 Gew.-%, und besonders bevorzugt 30 Gew.-%.

Das Gewichtsverhältnis der oben genannten Pflanzen bzw. deren Pflanzenteile bewegt sich optimal in einem Bereich von etwa 2:1 Salvia Miltiorrhizae zu Fructus Schizandrae.

Falls als Erdalkalimetallsalz Magnesium- und/oder Calciumcarbonat eingesetzt wird, beträgt deren Anteil an der Gesamtzusammensetzung 1-20 Gew.-%, bevorzugt 5-15 Gew.-%, und besonders bevorzugt 10 Gew.-%. Als Quelle für das Magnesium- und/oder Calciumcarbonat wird bevorzugt Dolomitkalk verwendet. Bezüglich der Eigenschaften und Herkunft von Dolomitkalk wird auf die obigen Ausführungen verwiesen. Für weitere Informationen über den erfindungsgemäß eingesetzten Dolomitkalk wird auf die Internet-Adresse: htip://www.dolomitwerk.de des Schöndorfer Dolomitwerks bzw. das öffentlich zugängliche Datenblatt Nr. 30xx.05 verwiesen.

Die erfindungsgemäße Zusammensetzung liegt vorzugsweise in Form einer pharmazeutischen Zusammensetzung vor. In diesem Fall enthält die pharmazeutische Zusammensetzung die wie oben angegebenen arzneilich wirksamen Bestandteile in den angegebenen Mengen und zusätzlich einen oder mehrere pharmazeutische Träger- oder Hilfsstoffe.

Vorzugsweise liegt die Zusammensetzung dann in Form eines in Kapseln abgefüllten Granulats, in Form eines Konzentrats zur Auflösung in Wasser oder in Form von aus Granulat verpressten Tabletten vor. Bezüglich Einzelheiten, wie solche Granulate, Extrakte etc. herstellbar sind, wird auf die unten angegebenen Informationen verwiesen.

Die Zusammensetzung kann in Snap-Fit-Hartgelatinekapseln enthalten sein, ebenso wie in Weichgelatinekapseln, die aus Gelatine und einem Weichmacher, wie beispielsweise Glycerol oder Sorbitol hergestellt sind. Die Kapseln können die wirksamen Verbindungen in fester oder flüssiger Form enthalten, die mit Füllstoffen, wie beispielsweise Lactose, Bindemitteln wie beispielsweise Stärken und/oder Gleitmitteln, wie beispielsweise Talkum oder Magnesiumstearat und wahlweise Stabilisatoren vermischt sein können.

In Weichgelatinekapseln werden die wirksamen Verbindungen vorzugsweise gelöst oder in geeigneten Flüssigkeiten suspendiert, wie beispielsweise in gepufferter Salzlösung, eingesetzt. Zusätzlich können Stabilisatoren zugesetzt werden.

Zusätzlich zur Verabreichung in flüssiger Form, beispielsweise in einer Gelatinekapsel oder einem anderen geeigneten Träger, können die pharmazeutischen Zubereitungen geeignete Trägerstoffe zur Erleichterung der Verarbeitung der wirksamen Verbindungen enthalten. Somit können pharmazeutische Zubereitungen zur oralen Anwendung durch Extrahieren der Pflanzenteile mit einem geeigneten Lösungsmittel, wie es dem Fachmann bekannt ist, Herstellen eines Trockenextraktes, wahlweise Vermahlen des sich ergebenden Extraktes und Verarbeiten des Extraktes zu Granulaten nach Zusatz von geeigneten Hilfsmitteln gewonnen werden. Die so gewonnenen Granulate können direkt in Kapseln abgefüllt oder zu Tabletten oder Drageekernen verpresst werden.

Geeignete Trägerstoffe sind insbesondere Füllmittel wie beispielsweise Zucker, beispielsweise Lactose oder Saccharose, Mannitol oder Sorbitol, Cellulosezubereitungen und/oder Calciumphosphate, beispielsweise Tricalciumphosphat oder Calciumhydrogenphosphat, ebenso wie Bindemittel, wie beispielsweise Stärke, unter Verwendung von beispielsweise Maisstärke, Weizenstärke, Reisstärke, Kartoffelstärke, Gelatine, Traganth, Methylcellulose, Hydroxypropylmethylcellulose, Natriumcarboxymethylcellulose und/oder Polyvinylpyrrolidon. Falls dies erwünscht ist, können Sprengmittel bzw. Desintegrationsmittel zugesetzt werden, wie beispielsweise die oben erwähnten Stärken und ebenfalls Carboxymethyl-Stärke, vernetztes Polyvinylpyrrolidon, Agar oder Alginsäure oder Salze hiervon, wie beispielsweise Natriumalginat.

Hilfsstoffe sind vor allem Fließregulierungsmittel und Gleitmittel, beispielsweise Siliciumdioxid, Talkum, Stearinsäure und Salze hiervon, wie beispielsweise Magnesiumstearat oder Calciumstearat und/oder Polyethylenglycol.

Drageekerne oder Tabletten werden mit geeigneten Beschichtungen bereitgestellt und falls dies erforderlich ist, sind sie magensaftresistent. Zu diesem Zweck können konzentrierte Zuckerlösungen verwendet werden, die wahlweise Gummi arabicum, Talkum, Polyvinylpyrrolidon, Polyethylenglycol und/oder Titandioxid, Lacklösungen und geeignete organische Lösungsmittel oder Lösungsmittelgemische enthalten. Um Beschichtungen bzw. Deckschichten zu erzeugen, die gegenüber Magensäure resistent sind, werden Lösungen geeigneter Cellulosezubereitungen, wie beispielsweise Acetylcellulosephthalat oder Hydroxypropylmethyl-Cellulosephthalat verwendet. Bevorzugt werden magensaftresistente Überzüge der Eudragit^{®} Reihe verwendet. Farbstoffe oder Pigmente können den Tabletten oder Drageebeschichtungen zugesetzt werden, beispielsweise zur besseren Identifizierung.

Die erfindungsgemäße Zusammensetzung liegt insbesondere als pharmazeutische Zusammensetzung, diätetisches Lebensmittel oder als Nahrungsergänzungsmittel vor. Als solches unterliegt die Einnahme der Inhaltstoffe der Zusammensetzungen keinerlei gesetzlichen Beschränkungen, so dass die Zusammensetzung auch im Lebensmittelbereich eingesetzt werden kann.

Gemäß einer bevorzugten Ausführungsform liegen die erfindungsgemäßen pflanzlichen Bestandteile in Granulatform vor, wobei das Gewichtsverhältnis von pflanzlichem Ausgangsmaterial zu Granulat von 10:1 bis 1:1, bevorzugt ungefähr 5:1 beträgt. Das Gewichtsverhältnis von ursprünglich eingesetzten Pflanzenteilen zu Granulat wird maßgeblich vom Extraktionsverfahren der ersteren zur Herstellung eines Trockenextraktes bestimmt. Unter bestimmten Umständen kommt auch die Direktverarbeitung der pflanzlichen Ausgangsmaterialien in Betracht, z.B. nach Vermahlen der pflanzlichen Ausgangsstoffe, dies stellt jedoch eine weniger bevorzugte Alternative dar.

Eine besonders bevorzugte Zusammensetzung der vorliegenden Erfindung enthält als arzneilich wirksame Bestandteile:

| | | |
|---|---|---|
| a) | Salvia Miltiorrhizae oder Pflanzenteile hiervon | 60 Gew.-% |
| b) | Fructus Schizandrae oder Pflanzenteile hiervon | 30 Gew.-% |
| c) | Dolomitkalk | 10 Gew.-% |

Zusätzlich können, wie oben angesprochen, noch weitere Bestandteile enthalten sein, z.B. Hilfsstoffe etc.

In einem zweiten Aspekt betrifft die Erfindung die Verwendung der wie oben definierten Zusammensetzung zur Behandlung von Schlafapnoe, krankhaften Formen des Schnarchens von durch Schnarchen bedingter Schlafstörungen und die Verwendung zur Herstellung eines Nahrungsergänzungsmittels oder eines diätetischen Lebensmittels.

Gemäß einer bevorzugten Ausführungsform enthält die Zusammensetzung die pflanzlichen Bestandteile in Granulatform und wird in einer Menge von 1-3 g, vorzugsweise ungefähr 2 g/Tag (arzneilich wirksame Bestandteile) einem erwachsenen Patienten verabreicht.

In einem dritten Aspekt betrifft die Erfindung ein Verfahren zur Herstellung einer wie oben definierten Zusammensetzung, das folgende Schritte umfasst:
a) Bereitstellen von Salvia Miltiorrhizae oder Pflanzenteilen hiervon, Fructus Schizandrae oder Pflanzenteilen hiervon und Magnesium- und/oder Calciumcarbonat;
b) Zerkleinern der pflanzlichen Bestandteile in einer geeigneten Vorrichtung;
c) Extrahieren der in b) zerkleinerten, pflanzlichen Bestandteile mit einem geeigneten Lösungsmittel;
d) Entfernen des Lösungsmittels zur Herstellung eines Trockenextraktes;
e) Mischen des in Schritt d) gewonnenen Trockenextraktes mit Magnesium- und/oder Calciumcarbonat und Verarbeiten der Mischung zu einer verabreichbaren Zusammensetzung.

Der Fachmann auf dem Gebiet ist jederzeit dazu in der Lage die Herstellungsbedingungen zu variieren, um zu anwendbaren Zusammensetzungen zu gelangen. Einschlägige Vorschriften sind insbesondere im DAB 2004 oder EAB 4. Ausgabe, 7. Nachtrag zu finden. Weitere einschlägige Fachinformationen sind in Fachbüchern der pharmazeutischen Technologie enthalten, z.B. "Pharmazeutische Technologie", Rudolf Voigt, 9., völlig überarb. Auflage, oder *"*Remington's Pharmaceutical Sciences", Mack Publishing Co., Easton, PA, 18. Ausgabe.

In einer bevorzugten Ausführungsform ist das Lösungsmittel Wasser und erfolgt das Extrahieren der pflanzlichen Bestandteile durch Kochen unter Niederdruck.

In einer Variante des Verfahrens werden aus den hergestellten Trockenextrakten jedes pflanzlichen Ausgangsmaterials jeweils Granulate hergestellt, die nachfolgend miteinander und mit den Erdalkalimetallsalzen vermischt werden, oder die Trockenextrakte werden mit den Salzen zunächst vermischt und dann granuliert.

Nachfolgend wird die Mischung vorzugsweise in Kapseln abgefüllt oder zu Tabletten verpresst.

Insbesondere werden in der vorliegenden Erfindung die folgenden Schritte berücksichtigt, die üblicherweise zur Herstellung von Pflanzenkonzentraten verwendet werden.

### 1. Überprüfung der Ausgangsmaterialien

Jede Charge der pflanzlichen Ausgangsmaterialien wird einer Quarantäne unterzogen, inspiziert und es wird ihr nach Eingang eine Ziffer zugeordnet. Nach Durchlaufen der Inspektion bezüglich der Pflanzenqualität, Pflanzenidentifizierung, der Schwermetallkonzentration, dem Vorhandensein von Käfern und anderer verunreinigender Stoffe und in speziellen Fällen der aktiven Inhaltsstoffe, werden die pflanzlichen Ausgangsmaterialien aus der Quarantäne entfernt und in den Speicherraum überführt. Die Materialien werden dann von Hand gereinigt und alle äußeren Materialien werden entfernt, um die weitere Verarbeitung vorzubereiten.

### 2. Extraktion

Die Zutaten für eine vorgegebene Zubereitung werden vereinigt und in einem großen Gefäß aus rostfreiem Stahl angeordnet, das mit Wasser befüllt ist und werden dann mit einer Wasseraufreinigungseinheit vor Ort behandelt. Die Kräuter werden für eine vorherbestimmte Zeitspanne eingeweicht und das Wasser wird auf eine voreingestellte Temperatur erhitzt. Die Kräuter werden für eine optimale Zeitspanne gekocht, um die meisten Inhaltstoffe zu extrahieren, ohne dabei überzukochen und die Inhaltsstoffe zu zerstören. Die Kochdauer und Temperaturen sind für jedes Produkt verschieden. In verschiedenen Zeitintervallen wird das Volumen und die Temperatur der Lösung aufgezeichnet. Zu Beginn des Heizprozesses werden ätherische Öle durch ein Wiedergewinnungssystem, das auf jedem Gefäß angebracht ist, gesammelt. Diese Öle werden während des Granulierprozesses wieder in das Produkt überführt.

### 3. Konzentration

Wenn die optimale Extraktionskonzentration erreicht ist oder wenn das eingesetzte Ausgangsmaterial erschöpft ist, werden die Kräuter aus dem Extraktionsgerät entfernt und die Flüssigkeit wird direkt in das Kondensiergerät gepumpt. Hier wird es durch Eindampfen in einem relativen Vakuum bei niedriger Temperatur kondensiert.

### 4. Granulierung

Nach Vakuumverdampfung wird die eingedickte Flüssigkeit in einen Vakuumtrockner und in eine Granulierkammer eingepumpt. Zu diesem Zeitpunkt wird ein Basispulver entweder aus Kartoffelstärke oder ein Pulver der pflanzlichen Ausgangsmaterialien in die Kammern gesprüht. Dieses Material vermischt sich mit dem Flüssigkeitsspray (unter Zusatz von Methylcellulose), so dass sich Granulate bilden. Zum Ende dieses Verfahrens hin werden die in der Extraktionsphase gesammelten ätherischen Öle in die Kammer eingebracht und von den Granulaten absorbiert. Alternativ kann der Extrakt als Trockenextrakt vorliegen und alleine/vermischt mit Stärke, mit Wasser (wahlweise + Methylcellulose) granuliert werden.

### 5. Verpackung

Die Granulate werden dann verpackt und in (recyclebare) Kunststoffflaschen eingeschweißt. Alle Verfahren nach der Extraktion werden in einer Rein-RaumAtmosphäre durchgeführt.
Grundsätzlich sind folgende Erwägungen bezüglich der Konzentration der Pflanzenkonzentrate in den Granulaten von Bedeutung:
Üblicherweise werden 250-500 g pflanzliche Ausgangsmaterialien zur Erzeugung von 100 g Konzentrat verwendet. Dies ergibt ein Konzentrationsverhältnis von 2,5:1 bis 5:1 abhängig von der speziellen Formel oder dem einzelnen pflanzlichen Ausgangsmaterial. Die meisten Formeln bewegen sich im Bereich von 3:1 bis 4:1.

Obwohl die meisten durch das obige Verfahren hergestellten Produkte Konzentrate sind, werden einige Ausgangsstoffe auch nicht konzentriert verwendet, beispielsweise Mineralstoffe, da diese nicht weiter aufkonzentriert werden können. Diese Bestandteile werden zu feinen Pulvern vermahlen und dann den Zusammensetzungen beigefügt.

Bezüglich der Dosierung werden in der chinesischen Medizin klassischer Weise 10 g der unmodifizierten Ausgangsformulierung dem Patienten verabreicht. Die Dosierung der Konzentrate beträgt ebenfalls ca. 10 g pro Tag, wobei Einzeldosen von ungefähr 3 g pro Tag (3 mal) bevorzugt werden.

Die Erfindung wird nachfolgend anhand eines Beispieles und der beigefügten Figuren veranschaulicht.

In den Figuren zeigt:
- Fig. 1:: die Schnarchentwicklung von 12 Probanden an den Tagen 0-5 nach Einnahme einer erfindungsgemäßen Zusammensetzung (Eigenbewertung);
- Fig. 2:: die Schnarchentwicklung der 12 Probanden gesamt;
- Fig. 3:: die Partnereinschätzung der Schnarchentwicklung der Probanden aus Fig. 1 und 2;
- Fig. 4:: die Partnereinschätzung der Schnarchentwicklung der 12 Probanden gesamt;
- Fig. 5:: die Entwicklung der Erholung des Schlafes nach Einnahme einer erfindungsgemäßen Zusammensetzung (Eigenbewertung) bei 12 Probanden;
- Fig. 6:: die Entwicklung der Erholung des Schlafes gesamt.

### Beispiel:

Die Wirksamkeit der Zusammensetzung konnte in einer Studie gezeigt werden. Dabei wurde eine Zusammensetzung mit folgenden Inhaltsstoffen und Herstellungsverfahren eingesetzt:
Als Ausgangsmaterialien werden die Bestandteile
   a) Salvia Miltiorrhizae oder Bestandteile hiervon 60 Gew.-%
   b) Fructus Schizandrae oder Bestandteile hiervon 30 Gew.-%
   c) Dolomitkalk 10 Gew.-%
   bereitgestellt.

Die pflanzlichen Bestandteile (Rohdrogen) werden zunächst auf Verunreinigungen (z.B. Pestizide, mikrobielle Verunreinigungen) überprüft. Danach werden sie durch eine übliche Vorrichtung zerkleinert.

Die zerkleinerten Bestandteile werden mit Wasser vermischt und unter Niederdruck gekocht. Dabei werden die flüchtigen ätherischen Öle aufgefangen und im nachfolgenden Schritt der Granulierung wieder in die Zusammensetzung mit aufgenommen.

Danach wird Wasser abgedampft und der Trockenextrakt mit wenig Wasser und etwas Stärke (ca. 5-10 % G/G derselben Pflanze) versetzt und nach üblichen Verfahren granuliert. Das Granulat wird nachfolgend in Hartgelatinekapseln abgefüllt.

In diesem Beispiel entspricht das Gewichts-Verhältnis von Rohdroge zu Granulat ca. 5:1.

Den Patienten wird eine Anzahl Kapseln verabreicht, die etwa 2 g wirksamer Zusammensetzung entspricht. Die Verabreichung erfolgt etwa 30 min. vor dem Schlafengehen.

Bei den Patienten mit Apnoe und Schnarchen wurde dann über einen Zeitraum von einer Woche die Ausprägung des nächtlichen Schnarchens und die Erholsamkeit des Schlafes vor bzw. während der Behandlung mit der Zusammensetzung erfragt.

Die Befragung erfolgte hinsichtlich folgender Parameter:
- Ausprägung des nächtlichen Schnarchens;
- Mundtrockenheit nach dem Erwachen;
- Erholsamkeit des Schlafes;
- Müdigkeit und Abgespanntheit während des folgenden Tages;
- Atempausen während des Schlafens;
- Verträglichkeit der Zusammensetzung.

Bezüglich der Verträglichkeit der Zusammensetzung ergaben sich keine Anhaltspunkte für unerwünschte Wirkungen.

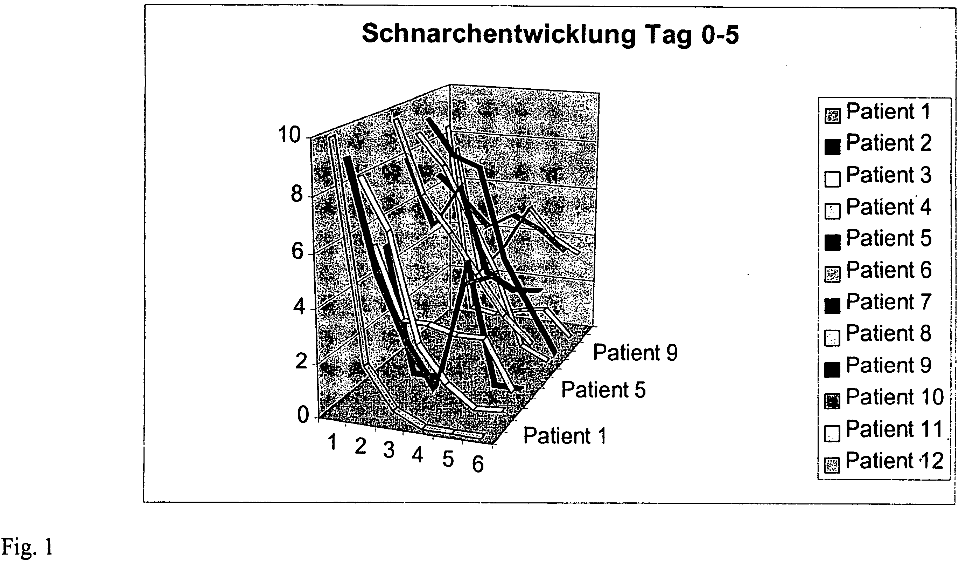

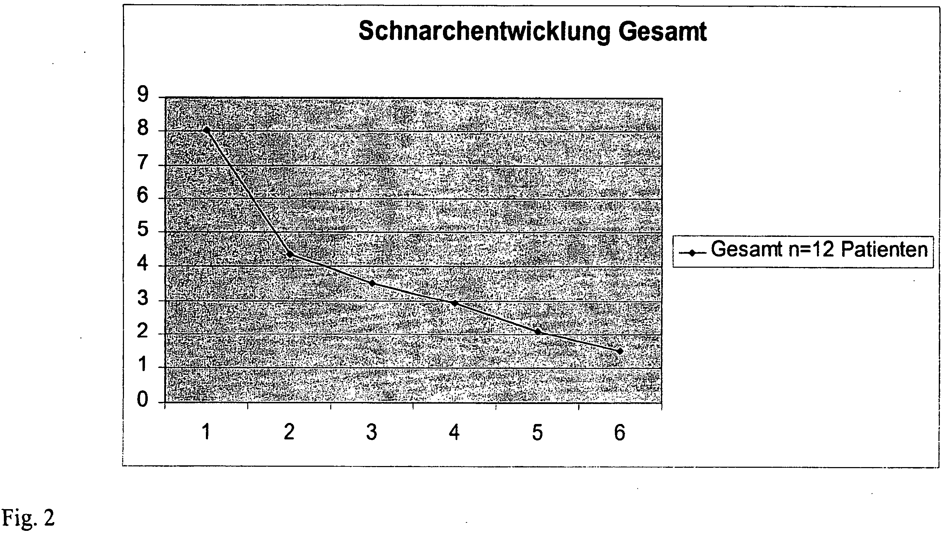

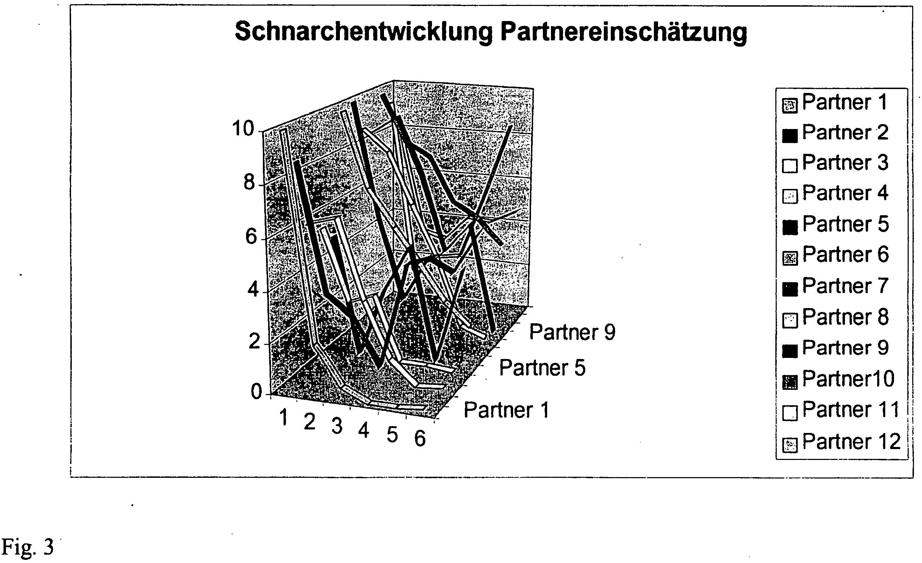

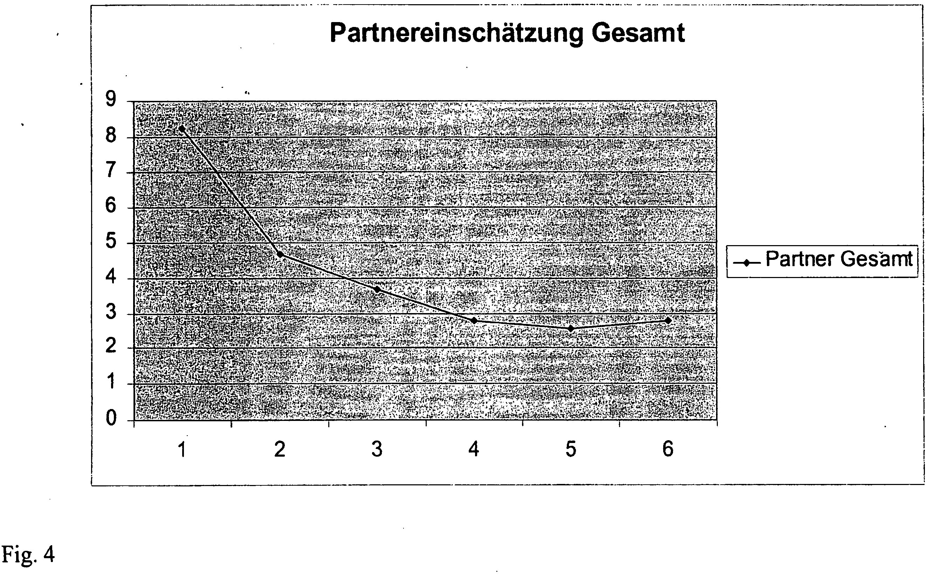

Die Beurteilung teilt sich in eine Selbsteinschätzung (Fig. 1, 2, 5 und 6) und eine Partnereinschätzung (Fig. 3 und 4) auf.

Deutlich ist als Ergebnis der Untersuchung und in allen graphischen Darstellungen die Abnahme der Intensität des Schnarchens und die verbesserte Erholung des Schlafes sichtbar. Ein Wert von 10 bedeutet sehr starkes Schnarchen bzw. sehr schlechte Erholsamkeit des Schlafes und ein Wert von 0 korreliert mit sehr geringem bzw. keinem Schnarchen und sehr guter Erholsamkeit des Schlafes.

Wie aus Fig. 1 ersichtlich ist konnte bei einigen Patienten (ca. 50%) binnen 6 Tagen die Schnarchentwicklung auf beinahe 0, d.h. kein oder nur sehr geringes Schnarchen, reduziert werden. Auch bei allen übrigen Patienten kam es zu einer deutlichen Abnahme des Schnarchens. Es ergab sich ein Durchschnittswert von 1,5 nach 6 Tagen Behandlung, bei einem Ausgangswert von 8.

Die Partnereinschätzung (Fig. 3) zeigte ebenfalls eine sehr deutliche Abnahme der Schnarchentwicklung. Auch hier wurde für ca. 50% der Patienten kein oder nur sehr geringes Schnarchen festgestellt. Auch bei den übrigen Patienten wurde eine deutliche Reduzierung des Schnarchens festgestellt. Lediglich bei zwei Patienten (5 und 11) ergab sich lediglich eine geringere Abnahme des Schnarchens. Auch hier zeigen die Durchschnittswerte eine deutliche Abnahme des Schnarchens von Wert 8,1 auf Wert 2,8.

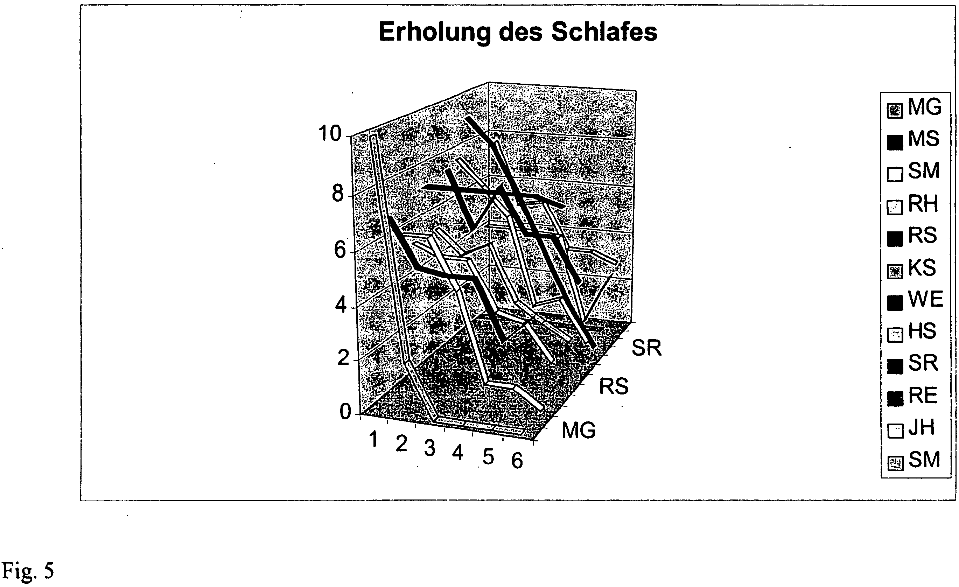

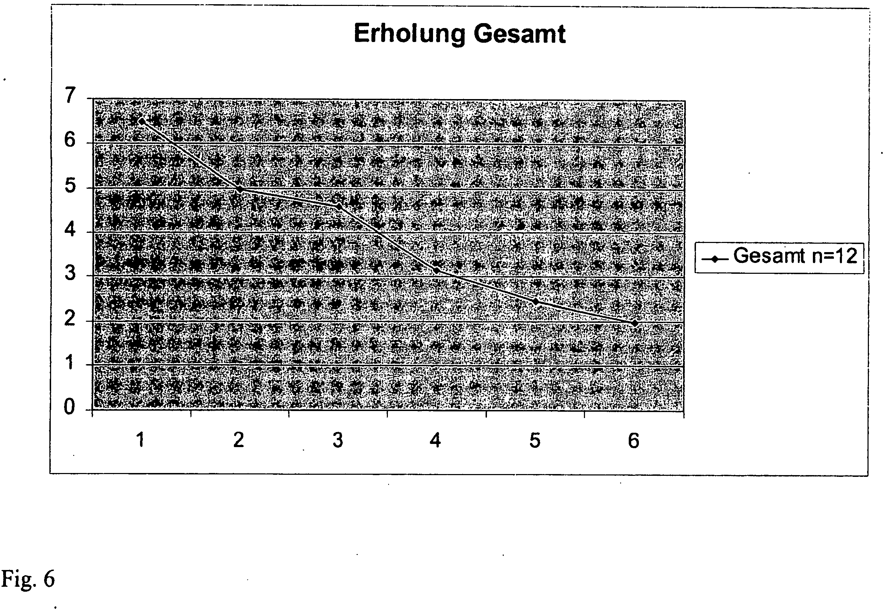

Zuletzt zeigte auch die Erholung des Schlafes eine deutliche Verbesserung nach Einnahme der erfindungsgemäßen Zusammensetzung (siehe Fig. 5 und 6).

## Patentansprüche

1. Zusammensetzung, umfassend als wirksame Bestandteile Salvia Miltiorrhizae oder Pflanzenteile hiervon, Fructus Schizandrae oder Pflanzenteile hiervon und ein oder mehrere Erdalkalimetallsalze, wobei die Erdalkalimetallsalze aus Calcium- und/oder Magnesiumsalzen, vorzugsweise Calcium- und/oder Magnesiumcarbonat, ausgewählt sind.

2. Zusammensetzung nach Anspruch 1, wobei der Anteil an Salvia Miltiorrhizae oder deren Pflanzenteile im Bereich von 40-80 Gew.-%, bevorzugt im Bereich von 50-70 Gew.-%, und besonders bevorzugt bei 60 Gew.-% liegt und/oder der Anteil an Fructus Schizandrae oder deren Pflanzenteile im Bereich von 10-50 Gew.-%, bevorzugt im Bereich von 20-40 Gew.-%, und besonders bevorzugt bei 30 Gew.-% liegt.

3. Zusammensetzung nach einem oder mehreren der vorhergehenden Ansprüche, wobei der Anteil an Magnesium- und/oder Calciumcarbonat 1-20 Gew.-%, bevorzugt 5-15 Gew.-%, und besonders bevorzugt 10 Gew.-% beträgt und/oder als Quelle für das Magnesium- und/oder Calciumcarbonat Dolomitkalk verwendet wird.

4. Zusammensetzung nach einem oder mehreren der vorhergehenden Ansprüche, wobei die Zusammensetzung in Form eines in Kapseln abgefüllten Granulats, in Form eines Konzentrats zur Auflösung in Wasser oder in Form von aus Granulat verpressten Tabletten vorliegt.

5. Zusammensetzung nach einem oder mehreren der vorhergehenden Ansprüche, wobei die Zusammensetzung eine pharmazeutische Zusammensetzung, ein diätetisches Lebensmittel oder ein Nahrungsergänzungsmittel ist.

6. Zusammensetzung nach einem oder mehreren der vorhergehenden Ansprüche, wobei die pflanzlichen Bestandteile in Granulatform vorliegen und das Gewichtsverhältnis von pflanzlichem Ausgangsmaterial zu Granulat von 10:1 bis 1:1, bevorzugt ungefähr 5:1 beträgt.

7. Zusammensetzung nach einem oder mehreren der vorhergehenden Ansprüche, wobei die Zusammensetzung aus folgendem besteht:
| | | |
|---|---|---|
| a) | Salvia Miltiorrhizae oder Pflanzenteile hiervon | 60 Gew.-% |
| b) | Fructus Schizandrae oder Pflanzenteile hiervon | 30 Gew.-% |
| c) | Dolomitkalk | 10 Gew.-% |

8. Zusammensetzung nach einem oder mehreren der Ansprüche 1-7 zur Verwendung zur Behandlung von Schlafapnoe, krankhaften Formen des Schnarchens und von durch Schnarchen bedingten Schlafstörungen.

9. Verwendung der Zusammensetzung nach einem oder mehreren der Ansprüche 1-7 zur Herstellung eines Nahrungsergänzungsmittels oder eines diätetischen Lebensmittels.

10. Verwendung nach Anspruch 8 oder 9, wobei die Zusammensetzung die pflanzlichen Bestandteile in Granulatform enthält und die Zusammensetzung in einer Menge von 1-3 g, vorzugsweise ungefähr 2 g/Tag an einen erwachsenen Patienten verabreicht wird.

11. Verfahren zur Herstellung einer Zusammensetzung nach einem oder mehreren der Ansprüche 1-7, das folgende Schritte umfasst:
a) Bereitstellen von Salvia Miltiorrhizae oder Pflanzenteilen hiervon, Fructus Schizandrae oder Pflanzenteilen hiervon und Magnesium- und/oder Calciumcarbonat;
b) Zerkleinern der pflanzlichen Bestandteile in einer geeigneten Vorrichtung;
c) Extrahieren der in b) zerkleinerten, pflanzlichen Bestandteile mit einem geeigneten Lösungsmittel;
d) Entfernen des Lösungsmittels zur Herstellung eines Trockenextraktes;
e) Mischen des in Schritt d) gewonnenen Trockenextraktes mit Magnesium- und/oder Calciumcarbonat und Verarbeiten der Mischung zu einer verabreichbaren Zusammensetzung.

12. Verfahren nach Anspruch 11, wobei das Lösungsmittel Wasser ist und das Extrahieren der pflanzlichen Bestandteile durch Kochen unter Niederdruck erfolgt.

13. Verfahren nach Anspruch 11 oder 12, wobei aus den hergestellten Trockenextrakten jeweils Granulate hergestellt werden, die nachfolgend vermischt werden, oder die Trockenextrakte mit Magnesium- und/oder Calciumcarbonat zunächst vermischt und dann granuliert werden und wahlweise die Mischung in Kapseln abgefüllt, zu Tabletten verpresst oder als Trinktabletten hergestellt wird.

## Claims

1. Composition, comprising as active components salvia miltiorrhizae or plant parts thereof, fructus schizandrae or plant parts thereof and one or more alkaline earth metal salts, where the alkaline earth metal salts are selected from calcium and/or magnesium salts, preferably calcium and/or magnesium carbonate.

2. Composition according to claim 1, where the amount of salvia miltiorrhizae or plant parts thereof is in the range of 40-80 weight-%, preferably in the range of 50-70 weight-%, and particularly preferred at 60 weight-% and/or wherein the amount of fructus schizandrae or plant parts thereof is in the range of 10-50 weight-%, preferably in the range of 20-40 weight-% and particularly preferred at 30 weight-%.

3. Composition according to one or more of the preceding claims, where the amount of magnesium and/or calcium carbonate is 1-20 weight-%, preferably 5-15 weight-%, and particularly preferred 10 weight-% and/or wherein dolomitic lime is used as a source for the magnesium and/or calcium carbonate.

4. Composition according to one or more of the preceding claims, where the composition is present in form of a granulate material filled in capsules, is present in form of a concentrate for dissolution in water or in form of granulate material compressed to tablets.

5. Composition according to one or more of the preceding claims, where the composition is a pharmaceutical composition, a dietetic food stuff or a nutritional supplement.

6. Composition according to one or more of the preceding claims, where the plant components are present in granulate form and the weight ratio of plant starting material to granulate is 10:1 to 1:1, preferably about 5:1.

7. Composition according to one or more of the preceding claims, where the composition consists of the following:
| | | |
|---|---|---|
| a) | Salvia miltiorrhizae or plant parts thereof | 60 weight-% |
| b) | Fructus schizandrae or plant parts thereof | 30 weight-% |
| c) | Dolomitic lime | 10 weight-% |

8. Compositions according to one or more of claims 1 - 7 for use in the treatment of sleep apnea, pathological forms of snoring and of sleep disturbances caused by snoring.

9. Use of a composition according to one or more of claims 1 - 7 for the manufacture of a nutritional supplement or a dietetic food stuff.

10. Use of claims 8 or 9, where the composition contains the plant components in granulate form and the composition is administered in an amount of 1-3 g, preferably 2 g/day to an adult patient.

11. Method for the manufacture of a composition according to one or more of claims 1 - 7 comprising the following steps:
a) providing salvia miltiorrhizae or plant parts thereof, fructus schizandrae or plant parts thereof and magnesium and/or calcium carbonate;
b) grinding the plant components in a suitable device;
c) extracting the plant components grinded in b) with a suitable solvent;
d) removing the solvent in order to prepare a dry extract;
e) mixing the dry extract obtained in step d) with magnesium and/or calcium carbonate and processing the mixture to a composition which is able to be administered.

12. The process of claim 11, where the solvent is water and where the extracting of plant components takes place by cooking under low pressure.

13. The process of claim 11 or 12, where granulates are made from the manufactured dry extracts, which are subsequently mixed, or where the dry extracts first are mixed with magnesium and/or calcium carbonate and then granulated and optionally where the mixture is filled in capsules, compressed to tablets or manufactured to drinking tablets.

## Revendications

1. Composition, comprenant comme éléments actifs de *Salvia Miltiorrhizae* ou ses composants végétaux, de *Fructus Schizandrae* ou ses composants végétaux et un ou plusieurs sels de métal alcalino-terreux, les sels de métal alcalino-terreux étant choisis parmi les sels de calcium et/ou de magnésium, préférentiellement le carbonate de calcium et/ou de magnésium.

2. Composition selon la revendication 1, la part de *Salvia Miltiorrhizae* ou de ses composants végétaux se situant entre 40 et 80 % en poids, préférentiellement entre 50 et 70 % en poids, et particulièrement préférentiellement à 60 % en poids et/ou la part de *Fructus Schizandrae* ou de ses composants végétaux se situant entre 10 et 50 % en poids, préférentiellement entre 20 et 40 % en poids, et particulièrement préférentiellement à 30 % en poids.

3. Composition selon l'une ou plusieurs des revendications précédentes, la part de carbonate de magnésium et/ou de calcium se situant entre 1 et 20 % en poids, préférentiellement entre 5 et 15 % en poids, et particulièrement préférentiellement à 10 % en poids et/ou de la chaux dolomitique étant utilisée comme source pour le carbonate de magnésium et/ou de calcium.

4. Composition selon l'une ou plusieurs des revendications précédentes, la composition se présentant sous la forme d'un granulat étant remplie dans des gélules, sous la forme d'un concentrat à dissoudre dans l'eau ou sous la forme de comprimés obtenus par agglomération du granulat.

5. Composition selon l'une ou plusieurs des revendications précédentes, la composition étant une composition pharmaceutique, un aliment diététique ou un complément alimentaire.

6. Composition selon l'une ou plusieurs des revendications précédentes, les composants végétaux se présentant sous la forme de granulat et le rapport de poids entre le matériau végétal de départ et le granulat étant de 10:1 à 1:1, préférentiellement de 5:1.

7. Composition selon l'une ou plusieurs des revendications précédentes, la composition étant constituée comme suit :
| | | |
|---|---|---|
| a) | *Salvia Miltiorrhizae* ou ses composants végétaux | 60 % en poids |
| b) | *Fructus Schizandrae* ou ses composants végétaux | 30 % en poids |
| c) | Chaux dolomitique | 10 % en poids |

8. Composition selon l'une ou plusieurs des revendications 1 à 7 destinée à une utilisation pour le traitement de l'apnée du sommeil, de formes maladives du ronflement et de troubles du sommeil liés au ronflement.

9. Utilisation de la composition selon l'une ou plusieurs des revendications 1 à 7 pour la fabrication d'un complément alimentaire ou d'un aliment diététique.

10. Utilisation selon la revendication 8 ou 9, la composition contenant les composants végétaux sous la forme de granulat et la composition étant administrée à un patient adulte dans une quantité de 1 à 3 g, préférentiellement environ 2 g/jour.

11. Procédé de fabrication d'une composition selon l'une ou plusieurs des revendications 1 à 7, qui comprend les étapes suivantes :
a) préparation de *Salvia Miltiorrhizae* ou de ses composants végétaux, de *Fructus Schizandrae* ou de ses composants végétaux et de carbonate de magnésium et/ou de calcium ;
b) broyage des composants végétaux dans un dispositif adapté ;
c) extraction des composants végétaux broyés en b) avec un solvant adapté ;
d) retrait du solvant pour fabriquer un extrait sec ;
e) mélange de l'extrait sec obtenu à l'étape d) avec du carbonate de magnésium et/ou de calcium et transformation du mélange en une composition pouvant être administrée.

12. Procédé selon la revendication 11, le solvant étant de l'eau et l'extraction du composant végétal s'effectuant par cuisson basse pression.

13. Procédé selon la revendication 11 ou 12, des granulats étant fabriqués à partir des différents extraits secs fabriqués, qui sont ensuite mélangés, ou les extraits secs étant ensuite mélangés avec du carbonate de magnésium et/ou de calcium puis étant granulés et le mélange étant au choix rempli dans des gélules, aggloméré sous forme de comprimés ou fabriqué sous forme de comprimés à boire.
